# EUROPEAN PATENT SPECIFICATION

(11) **EP 4 333 741 B1**
(45) Date of publication and mention of the grant of the patent: **27.08.2025**
(21) Application number: 22723791.4
(22) Date of filing: 03.05.2022
(51) Int. Cl.: A61B 17/32, A61B 17/42, A61B 46/10, A61M 1/00, A61B 46/23, A61B 90/00, A61B 46/00

(54) **TISSUE RESECTION SYSTEMS INCLUDING FLUID OUTFLOW MANAGEMENT**
GEWEBERESEKTIONSSYSTEME MIT FLÜSSIGKEITSAUSFLUSSVERWALTUNG
SYSTÈMES DE RÉSECTION DE TISSU À COMPOSANT DE GESTION DE SORTIE DE FLUIDE

(30) Priority: 06.05.2021 US 202163185093 P
(43) Date of publication of application: 13.03.2024
(73) Proprietor: Covidien LP, Mansfield, MA 02048 (US)
(72) Inventor: PRIOR, Scott J., North Haven, Connecticut 06473 (US); BEGG, Nikolai D., Woburn, Massachusetts 01801 (US); PICKERING, Chad A., Woburn, Massachusetts 01801 (US); RAJAGOPALAN MOHAN, Arvind, Woburn, Massachusetts 01801 (US); WOOD, Timothy J., Woburn, Massachusetts 01801 (US)
(74) Representative: Maschio & Soames IP Ltd
(86) International application number: PCT/IB2022/054074
(87) International publication number: WO 2022/234449

(56) References cited:
- WO-A2-2020/109804
- US-A- 6 162 187
- US-A1- 2006 047 185
- US-A1- 2009 270 898
- US-A1- 2012 172 888
- US-A1- 2012 289 894
- US-A1- 2014 112 841
- US-A1- 2014 303 551
- US-A1- 2019 255 312
- US-A1- 2020 179 576
- US-B1- 6 652 481
- US-B2- 10 159 790

## Description

### TECHNICAL FIELD

The present disclosure relates generally to surgical systems and, more particularly, to tissue resection systems including fluid outflow management.

### BACKGROUND

Surgical procedures, such as tissue resection procedures, may be performed endoscopically within an organ, such as a uterus, by inserting an endoscope (or hysteroscope) into the uterus and passing a tissue resection device through the endoscope and into the uterus. With respect to such hysteroscopic tissue resection procedures, it often is desirable to distend the uterus with a fluid, for example, saline, sorbitol, or glycine. The inflow and outflow of the fluid during the procedure maintains the uterus in a distended state and flushes tissue and other debris from within the uterus to maintain a visible working space.

US 10 159 790 B2 discloses a surgical fluid management system comprising:a surgical drape, a collection container and a fluid outflow tube coupled to the surgical drape and the collection container.

US 2014/112841 A1 discloses a blood or urine sampling device having a soluble membrane to expose an outlet after a predetermined amount of time such that it can flow to a collection chamber.

### SUMMARY

The invention is defined by claim 1. Further embodiments of the invention are defined by the dependent claims.

Provided in accordance with aspects of the present disclosure is a tissue resection system having improved fluid outflow management that lessens the start-up time of the tissue resection system and/or minimizes poor tissue resection performance (e.g., inadequate suction for fluid and tissue removal) due to low outflow pressure.

In an aspect of the present disclosure, a surgical fluid management system includes a surgical drape, a collection container, a fluid outflow tube coupled to the surgical drape and the collection container for withdrawal of exudate from the surgical drape into the collection container, and a valve disposed within the fluid outflow tube. The valve has a closed position for preventing flow into the collection container from the surgical drape and an open position for enabling flow through the surgical drape into the collection container.

The valve may be a stopcock valve.

The surgical fluid management system may further include a control console including a vacuum pump assembly and a vacuum line coupled to the vacuum pump assembly and the collection container for providing suction through the fluid outflow tube. The control console may control the valve.

The surgical drape may include a body defining a cavity therein, and a second end of the body may be coupled to the fluid outflow tube.

In another aspect of the present disclosure a surgical fluid management system includes a surgical drape including a body defining a cavity therein, a collection container, a fluid outflow tube coupled to the surgical drape and the collection container for withdrawal of exudate from the surgical drape into the collection container, and a membrane extending across the cavity of the surgical drape to close a fluid flow path between the surgical drape and the fluid outflow tube.

The membrane may be formed from a dissolvable or bioerodible polymer.

The surgical fluid management system may further include a control console including a vacuum pump assembly and a vacuum line coupled to the vacuum pump assembly and the collection container for providing suction through the fluid outflow tube.

In yet another aspect of the present disclosure, a surgical fluid management system includes an endoscope, a surgical drape, a first collection container, a second collection container, a first fluid outflow tube coupled to the endoscope and the first collection container for withdrawal of fluid from the endoscope into the first collection container, a second fluid outflow tube coupled to the surgical drape and the second collection container for withdrawal of fluid from the surgical drape into the second collection container, and a vacuum pump assembly including a vacuum line selector for controlling suction through the first and second fluid outflow tubes.

The surgical fluid management system may further include a first vacuum line coupled to the vacuum pump assembly and the first collection container for providing suction through the first fluid outflow tube, and a second vacuum line coupled to the vacuum pump assembly and the second collection container for providing suction through the second fluid outflow tube. The vacuum line selector may include a valve. In some aspects, the vacuum line selector is coupled to an actuator that is manually adjustable to open and close the first and second vacuum lines and, in other aspects, the vacuum line selector is motorized and programmed to automatically open and close the first and second vacuum lines.

In another aspect of the present disclosure, a surgical fluid management system includes an endoscope, a fluid source, a fluid inflow tube coupled to the endoscope and the fluid source for enabling delivery of fluid from the fluid source into the endoscope, a collection container, a fluid outflow tube coupled to the endoscope and the collection container for enabling withdrawal of fluid through the endoscope and into the collection container, and a pressure pump assembly including a pressure line selector for controlling pressure on the fluid source and in the collection container.

The surgical fluid management system may further include a first pressure line coupled to the pressure pump assembly and the fluid source for pressurizing fluid in the fluid source, and a second pressure line coupled to the pressure pump assembly and the collection container for drawing negative pressure through the collection container. The pressure line selector may include a valve for diverting pressure to the first or second pressure line. The pressure line selector may be motorized and programmed to automatically utilize either the first or second pressure line.

The surgical fluid management system may further include a vacuum pump assembly configured to establish negative pressure through the collection container. The pressure line selector may be configured to divert pressure into the second pressure line to aid the vacuum pump assembly in maintaining negative pressure in the collection container.

In still another aspect of the present disclosure, a tissue resection system includes a surgical instrument, a collection container, a fluid and debris outflow tube, a vacuum pump, and a vacuum line. The fluid and debris outflow tube is coupled to the surgical instrument and the collection container for withdrawal of fluid and debris through the surgical instrument and into the collection container. The vacuum pump assembly includes a vacuum pump and a vacuum monitoring assembly configured to monitor flow characteristics through the vacuum pump. The vacuum line is coupled to the vacuum pump assembly and the collection container for providing suction through the fluid and debris outflow tube.

The vacuum pump assembly may be a component of a control console. The control console may be configured to provide an alert when the vacuum monitoring assembly reads a threshold flow characteristic of the vacuum pump. The control console may prevent activation of the surgical instrument until the vacuum monitoring assembly reads a threshold flow characteristic of the vacuum pump. The threshold flow characteristic may be vacuum pressure.

The surgical instrument may include a handpiece and an end effector assembly releasably secured to the handpiece. The surgical instrument may include a valve disposed within the fluid and debris outflow tube. In some aspects, the valve is movable between a closed position when the vacuum monitoring assembly measures flow characteristics associated with air flow and an open position when the vacuum monitoring assembly measures flow characteristics associated with liquid flow. The valve may be a flow rate dependent valve. In other aspects, the valve is movable between a closed position when the end effector is not attached to the handpiece and an open position when the end effector is attached to the handpiece. The handpiece may include a communication receiver and the end effector assembly may include a communication device such that when the end effector assembly is attached to the handpiece, the communication device relays to the communication receiver that the end effector assembly is attached to the handpiece and the control console moves the valve to the open position. In some aspects, the valve is movable from a closed position when the surgical instrument is not activated for use and an open position when the surgical instrument is activated for use. The handpiece may include a drive mechanism therein, and the valve may be in electrical communication with the drive mechanism. The valve may be a motor-actuated valve or a pneumatically-actuated valve. In some aspects, the valve is coupled to an output interface of the handpiece. The valve may be a stopcock valve.

The handpiece of the surgical instrument may include a film disposed over and closing off an interface of the handpiece that connects with the end effector assembly, and when the end effector assembly is engaged with the handpiece, the film is opened.

The details of one or more aspects of the disclosure are set forth in the accompanying drawings and the description below. Other aspects, as well as features, objects, and advantages of the aspects described in this disclosure will be apparent from the description and drawings, and from the claims.

### BRIEF DESCRIPTION OF THE DRAWINGS

Various aspects and features of the present disclosure are described hereinbelow with reference to the drawings wherein like numerals designate identical or corresponding elements in each of the several views.
FIG. 1 is a perspective view of a tissue resection system in accordance with aspects of the present disclosure;
FIG. 2 is a schematic view of a control console of the tissue resection system of FIG. 1;
FIG. 3 is a schematic view of a surgical instrument of the tissue resection system of FIG. 1 in accordance with an aspect of the present disclosure;
FIG. 4 is a schematic view of a surgical instrument of the tissue resection system of FIG. 1 in accordance with another aspect of the present disclosure;
FIG. 5 is a schematic view of a surgical instrument of the tissue resection system of FIG. 1 in accordance with yet another aspect of the present disclosure;
FIG. 6 is a perspective view of a surgical instrument of the tissue resection system of FIG. 1 in accordance with another aspect of the present disclosure;
FIG. 7 is a perspective view of a handpiece of a surgical instrument of the tissue resection system of FIG. 1 in accordance with another aspect of the present disclosure;
FIG. 8 is a front view of a surgical drape of the tissue resection system of FIG. 1 in accordance with an aspect of the present disclosure;
FIG. 9 is a cross-sectional view of a surgical drape of the tissue resection system of FIG. 1 in accordance with another aspect of the present disclosure;
FIG. 10 is a perspective view of components of a tissue resection system in accordance with another aspect of the present disclosure; and
FIG. 11 is a perspective view of components of a tissue resection system in accordance with yet another aspect of the present disclosure.

### DETAILED DESCRIPTION

Aspects of the present disclosure will now be described in detail with reference to the drawing figures wherein like reference numerals identify similar or identical elements. Throughout this description, the term "proximal" refers to a portion of a structure, or component thereof, that is closer to a user, and the term "distal" refers to a portion of the structure, or component thereof, that is farther from the user.

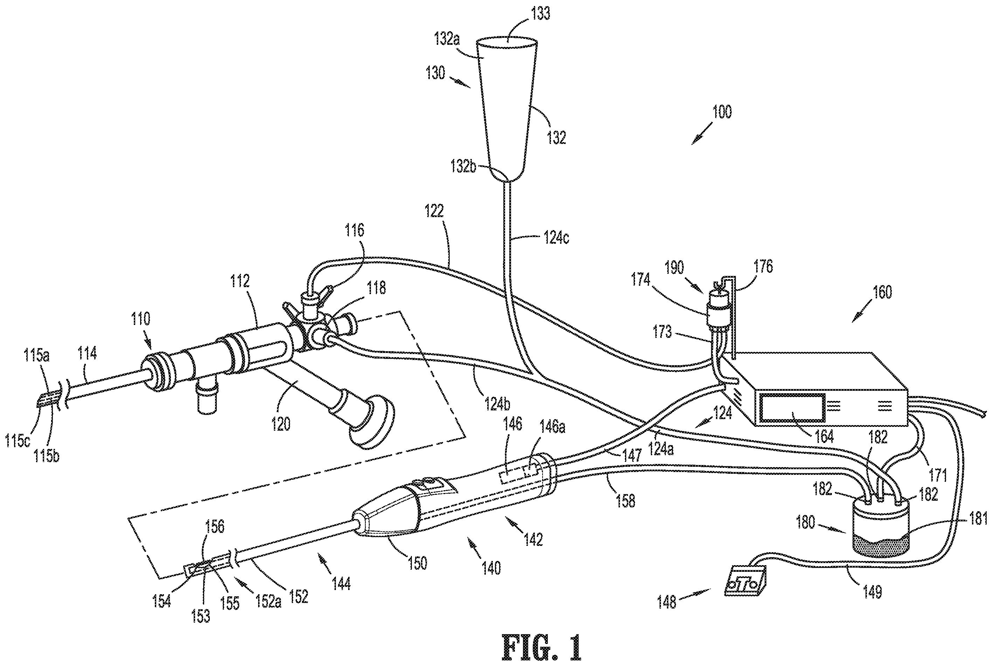

Referring now to FIG. 1, a tissue resection system 100 in accordance with aspects of the present disclosure is shown. The tissue resection system 100 generally includes an endoscope 110, a surgical drape 130, a surgical instrument 140, a control console 160, a collection container 180, and a fluid source 190, as well as associated tubing (e.g., for fluid flow) and cables (e.g., for power).

The endoscope 110 is described herein as a hysteroscope configured for use within the uterus of a female patient. However, other suitable endoscopes and/or fluid-delivery devices are also contemplated for use in the tissue resection system 100 of the present disclosure on or within different anatomical structures. The endoscope 110 includes a body 112 and an elongate tube 114 extending distally from the body 112. The body 112 includes an inflow valve 116, an outflow valve 118, and an arm 120 configured to connect to an imaging device (e.g., a camera) to capture images received via a visualization mechanism, e.g., optics (not shown), extending through the elongate tube 114. The elongate tube 114 defines a first channel 115a for fluid inflow, a second channel 115b that is shared between fluid outflow and instrument access, e.g., for the surgical instrument 140, and a third channel 115c housing the visualization mechanism (not shown). Alternatively, the first channel 115a is shared between fluid inflow and instrument access while the second channel 115b is dedicated to fluid outflow. The first channel 115a is coupled to the inflow valve 116 to enable the introduction of fluid through the endoscope 110 and into a patient. A fluid inflow tube 122 is coupled to the inflow valve 116 and the fluid source 190 for enabling the delivery of fluid from the fluid source 190 into the endoscope 110, and thus, ultimately into the patient. The second channel 115b is coupled to the outflow valve 118 to enable the withdrawal of fluid from the patient through the endoscope 110. A fluid outflow tube 124 is coupled to the outflow valve 118 and the collection container 180 for enabling the withdrawal of fluid from the patient into the endoscope 110 and into the collection container 180.

The fluid outflow tube 124 is also connected to the surgical drape 130 for collecting exudate (e.g., body fluid, tissue, etc.) from the patient into the collection container 180. The fluid outflow tube 124 is a y-shaped tube having a main line 124a coupled to the collection container 180 that splits into two branch lines 124b, 124c (also referred to herein as an endoscope line and a drape line, respectively), with the endoscope line 124b coupled to the inflow valve 116 of the endoscope 110 and the drape line 124c coupled to the surgical drape 130. Alternatively, the endoscope and drape lines 124b, 124c may separately connect to collection container 180.

The surgical drape 130 has a body 132 including first and second ends 132a, 132b and defining a cavity 133 therein. The body 132 may have a generally funnel shape such that the first end 132a has a larger dimension than the second end 132b. The first end 132a is configured for positioning beneath a patient and catching the exudate released from the patient, and the second end 132b is coupled to the drape line 124c of the fluid outflow tube 124, as described above.

The surgical instrument 140 is described herein as a tissue resecting device, however, other suitable surgical instruments are also contemplated for use in the system 100 of the present disclosure. The surgical instrument 140 generally includes a handpiece 142 and an end effector assembly 144 extending distally from the handpiece 142. The end effector assembly 144 is configured to releasably engage the handpiece 142. In aspects, the handpiece 142 is a reusable component and the end effector assembly 144 is a single-use disposable component. The handpiece 142 supports a drive mechanism 146 therein. The drive mechanism 146 includes a motor 146a that is operably coupled to the end effector assembly 144 to effect a function of the end effector assembly 144. The drive mechanism 146 is adapted to connect to the control console 160 via a cable 147 for powering and controlling the motor 146a. An actuator 148, shown in the form of a foot pedal, is coupled to the drive mechanism 146, via the control console 160, by a cable 149 for enabling the selective activation of the surgical instrument 140. Handswitches on the handpiece 142 are alternatively or additionally contemplated.

The end effector assembly 144 includes hub 150, a shaft 152 extending distally from the hub 150, and a cutting tool 154 extending through the shaft 152. The shaft 152 defines a window 153 through a side wall thereof at a distal end portion 152a of the shaft 152 to provide access to the cutting tool 154 which is rotatably and/or translatably disposed within the shaft 152 and operably coupled to the drive mechanism 146. The cutting tool 154 defines an opening 155 providing access to the interior thereof and may include serrated cutting edges 156 surrounding the opening 155, although other suitable cutting and/or shaving edge configurations are contemplated.

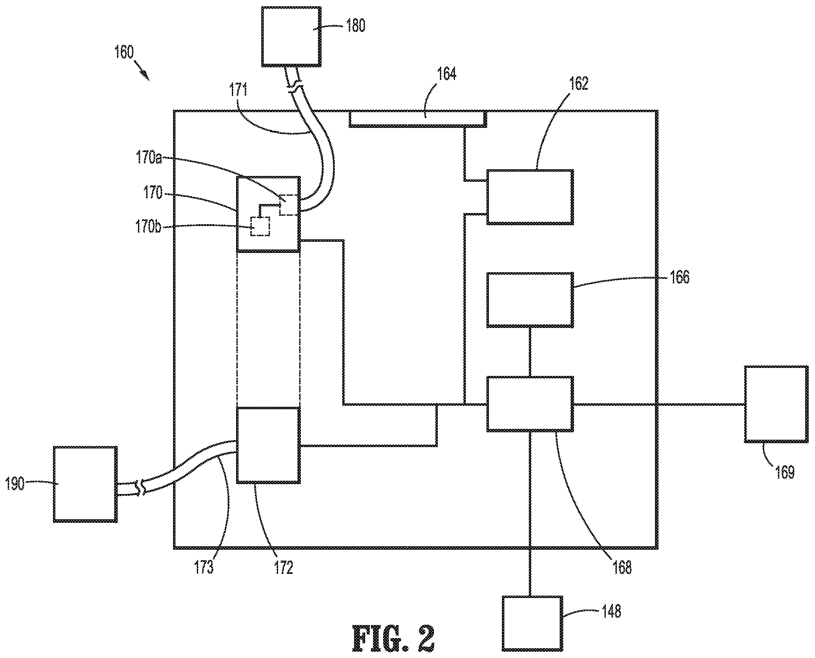

A fluid and debris outflow tube 158 is coupled to the cutting tool 154 and extends through the handpiece 142 to the collection container 180 for enabling the withdrawal of fluid (e.g., fluid from the fluid source 190, body fluids, etc.) and debris (e.g., resected tissue, etc.) suctioned from the patient through the cutting tool 154 and into the collection container 180. The fluid and debris outflow tube 158 is operably coupled with a vacuum pump assembly 170 (FIG. 2) of the control console 160 to enable suctioning of the fluid and debris through the cutting tool 154 and into the collection container 180.

As shown in FIG. 2, in conjunction with FIG. 1, the control console 160 includes a controller assembly 162 and a display 164. The controller assembly 162 is configured to control the display of information on the display 164 and may be configured to receive and process information inputted thereto (e.g., via a touch-screen of the display 164). The control console 160 supports a motor control assembly 166 therein that is configured to control the drive mechanism 146 of the surgical instrument 140, a power supply 168 configured to convert power from a main power supply 169 into suitable form for powering the control console 160, a vacuum pump assembly 170 configured to control suction through the endoscope 110, the surgical drape 130, and the surgical instrument 140, and, in aspects, a pressure pump assembly 172 configured to pressurize the fluid source 190 supplied to the endoscope 110. The components of the control console 160 include suitable hardware and may also include one or more processors and associated memories storing software to be executed by the processors to control the hardware components (although one or more centralized processors and/or memories may alternatively be provided).

A vacuum line 171 (e.g., vacuum tubing) is coupled to the vacuum pump assembly 170 and the collection container 180 for establishing negative pressure through the collection container 180 and thus, through the fluid outflow tube 124 and the fluid and debris outflow tube 158 to ultimately draw fluid and debris from the endoscope 110, the surgical drape 130, and the surgical instrument 140 into the collection container 180. A pressure line 173 is coupled to the pressure pump assembly 172 and the fluid source 190. The pressure line 173 includes a cuff 174 (FIG. 1) positioned around the fluid source 190 for pressurizing the fluid therein.

With continued reference to FIG. 1, the collection container 180 defines a chamber 181 therein and includes a plurality of ports 182 in fluid communication with the chamber 181. The fluid outflow tube 124 and the fluid and debris outflow tube 158 are coupled to the ports 182 to enable passage of fluid and debris into the chamber 181, and the vacuum line 171 is also coupled to one of the ports 182 to draw vacuum therethrough. In some aspects, the tissue resection system 100 includes a plurality of collection containers 181, and in certain aspects, the control console 160 includes a support (not shown) for supporting the collection container(s).

The fluid source 190 is a fluid bag containing a fluid, e.g., saline, sorbitol, or glycine, therein. It should be understood that other configurations of the fluid source 190 are envisioned. The fluid source 190 is connected to the fluid inflow tube 122 which, as described above, is coupled to inflow valve 116 of the endoscope 110 for enabling delivery of fluid from the fluid source 190 to the endoscope 110. The cuff 174 of the pressure line 173 is disposed around the fluid source 190 to pressurize the fluid therein and drive the fluid through the fluid inflow tube 122 and the endoscope 110 and, ultimately, into the patient. In some aspects, the tissue resection system 100 includes a plurality of fluid sources 190 and, in certain aspects, the control console 160 includes a support (e.g., support pole 176) for supporting the fluid source(s) 190. In aspects, the control console 160, the fluid source(s) 190, the collection container(s) 180, and/or other components (e.g., the actuator 148) are assembled together, such as, on a cart.

In use, the user activates the control console 160 to start up and prime the tissue resection system 100 for use. Upon activation, the vacuum pump assembly 170 establishes negative pressure through the collection container 180 to control suction through the endoscope 110, the surgical drape 130, and the surgical instrument 140. During start-up, the endoscope 110, the surgical drape 130, and the surgical instrument 140 may be open to the atmosphere (e.g., there is no or minimal fluid in the tissue resection system 100) which results in suctioning of air therethrough. This can lead to loss of suction or a delay in generating adequate vacuum pressure in the tissue resection system 100 which is needed to facilitate tissue removal and inhibit uterus collapse. In order to establish adequate vacuum pressure for optimal performance of the tissue resection system 100 and/or to minimize the start-up time for reaching a threshold vacuum pressure for use, the tissue resection system 100 includes one or more of the following fluid outflow management features.

As shown in FIG. 2, the vacuum pump assembly 170 includes a vacuum pump 170a and a vacuum monitoring assembly 170b configured for monitoring flow characteristics (e.g., vacuum pressure, flow rate, flow resistance, etc.) through the vacuum pump 170a. The vacuum pump assembly 170 may adjust the operating parameters of the vacuum pump 170a based on the flow characteristics (e.g., vacuum pressure readings) measured by the vacuum monitoring assembly 170b. The control console 160 is configured to provide an alert (e.g., a visual or audible alert) on the display 164 to the user when sufficient vacuum pressure is achieved (e.g., the vacuum monitoring assembly 170b reads a threshold vacuum pressure suitable for use) and the tissue resection system 100 is ready for use and/or that there is insufficient vacuum pressure and the tissue resection system 100 is not ready for use. In some aspects, the control console 160 is configured to prevent activation of the surgical instrument 140 (FIG. 1) until optimal flow characteristics (e.g., a threshold vacuum pressure) is achieved for use of the tissue resection system 100.

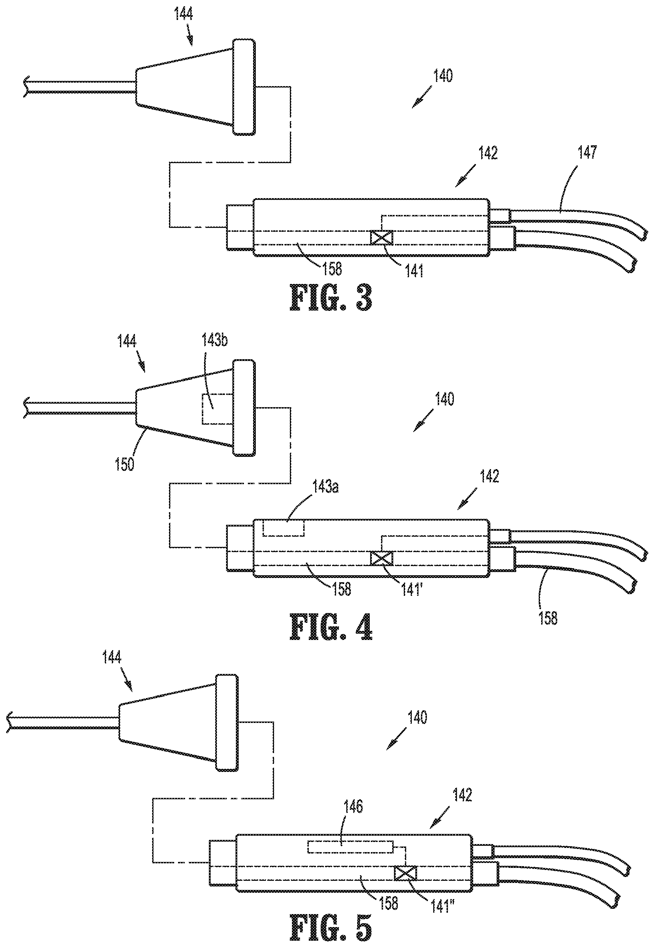

As shown in FIG. 3, the surgical instrument 140 includes a valve 141 to aid in establishing or maintaining adequate operating vacuum pressure in the tissue resection system 100 in accordance with an aspect of the present disclosure. The valve 141 is disposed within the handpiece 142 of the surgical instrument 140 and is coupled to the fluid and debris outflow tube 158 to control the flow of fluid and debris therethrough. The valve 141 is disposed within the flow path of the fluid and debris outflow tube 158 to selectively permit and inhibit flow therethrough and/or to control the flow rate therethrough. Accordingly, the valve 141 is movable between a closed position for inhibiting flow through the fluid and debris outflow tube 158 and an open position for enabling free flow through the fluid and debris outflow tube 158, and may include one or more partially open positions for enabling partial flow through the fluid and debris outflow tube 158.

In aspects, the valve 141 is a flow rate dependent valve that prevents or reduces flow through the fluid and debris outflow tube 158 when air is sensed in the tissue resection system 100 and adjusts to enable full flow therethrough once liquid is sensed in the tissue resection system 100. In some aspects, the flow rate through the valve 141 is controlled mechanically therein by, for example, a diaphragm, a flap, or a membrane disposed within the valve 141 that moves between the open and closed positions in response to the amount of vacuum pressure within the fluid and debris outflow tube 158. In other aspects, the valve 141, e.g., as a solenoid or other electrically-controlled valve, is controlled by the control console 160 (FIG. 2) through an electrical connection via the cable 147 connecting the handpiece 142 to the control console 160. The control console 160 moves the valve 141 between the open and closed positions in response to the flow characteristics read by the vacuum monitoring assembly 170b (FIG. 2). The valve 141 is moved to the closed position or a partially open position when the vacuum monitoring assembly 170b reads flow characteristics associated with air flow and is moved to the open position when the vacuum monitoring assembly 170b reads flow characteristics associated with liquid flow.

As shown in FIG. 4, the surgical instrument 140 includes a valve 141' to aid in establishing or maintaining adequate operating vacuum pressure in the tissue resection system 100 in accordance with another aspect of the present disclosure. The valve 141' is disposed within the handpiece 142 of the surgical instrument 140 and is coupled to the fluid and debris outflow tube 158 to control the flow of fluid and debris therethrough. The valve 141' is disposed within the flow path of the fluid and debris outflow tube 158 to selectively permit and inhibit flow therethrough and/or to control the flow rate therethrough. Accordingly, the valve 141' is movable between a closed position for inhibiting flow through the fluid and debris outflow tube 158 and an open position for enabling free flow through the fluid and debris outflow tube 158.

The valve 141' is in the closed position when the end effector assembly 144 is not attached to the handpiece 142 and is moved to the open position when the end effector assembly 144 is attached to the handpiece 142. The handpiece 142 includes a communication receiver 143a, e.g., an RFID reader, disposed therein and the end effector assembly 144 includes a communication device 143b, e.g., an RFID tag, in the hub 150 thereof for storing information regarding the end effector assembly 144, such as, for example, identifying information, use setting information, etc. Upon attachment of the end effector assembly 144 with the handpiece 142, the communication device 143b is disposed in contact with or sufficient proximity relative to the communication receiver 143a to enable the communication receiver 143a to read information from the communication device 143b and relay the same to the control console 160. Specifically, the communication device 143b relays that the end effector assembly 144 is attached to the handpiece 142 and the control console 160 opens the valve 141' to establish flow through the fluid and debris outflow tube 158. Alternatively, the valve 141' may be disposed in a distal end of the handpiece 142 such that the valve 141' is opened manually upon attachment of the end effector assembly 144 to the handpiece 142, e.g., via a portion of the end effector assembly 114 physically contacting (directly or indirectly) and manipulating (directly or indirectly) the valve 141'.

During start-up, the end effector assembly 144 remains separate from the handpiece 142 so that the valve 141' remains closed and the flow of ambient air through the handpiece 142 is prevented. The end effector assembly 144 is attached to the handpiece 142 after the tissue resection system 100 is primed and ready for use (e.g., as one of the last steps of the start-up process to minimize the amount of ambient air entered in the tissue resection system 100).

As shown in FIG. 5, the surgical instrument 140 includes a valve 141" to aid in establishing or maintaining adequate operating vacuum pressure in the tissue resection system 100 in accordance with another aspect of the present disclosure. The valve 141" is disposed within the handpiece 142 of the surgical instrument 140 and is coupled to the fluid and debris outflow tube 158 to control the flow of fluid and debris therethrough. The valve 141" is disposed within the flow path of the fluid and debris outflow tube 158 to selectively permit and inhibit flow therethrough and/or to control the flow rate therethrough. Accordingly, the valve 141" is movable between a closed position for inhibiting flow through the fluid and debris outflow tube 158 and an open position for enabling free flow through the fluid and debris outflow tube 158.

The valve 141" is in the closed position when the surgical instrument 140 is not in use and moves to the open position when the surgical instrument 140 is activated for use (e.g., by depressing the foot pedal 148 (FIG. 1)). The valve 141" is electrically coupled to the drive mechanism 146 of the handpiece 142 such that upon activation of the surgical instrument 140, the drive mechanism 146 sends a signal to open the valve 141". In some aspects, the valve 141" is a motor-actuated valve, in some other aspects, the valve 141" is a pneumatically-actuated valve, and in still other aspects, the valve 141" is an electrically-actuated valve.

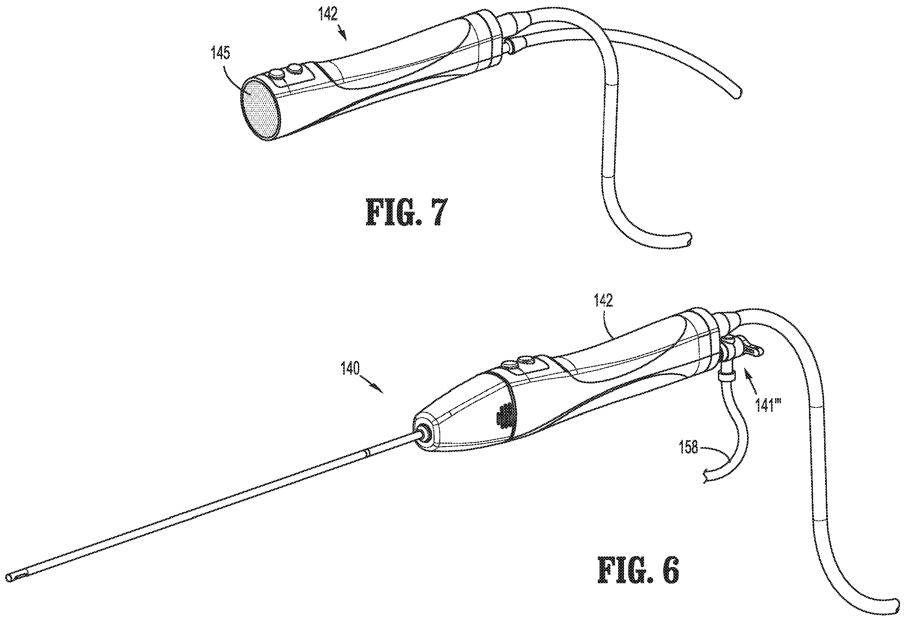

As shown in FIG. 6, the surgical instrument 140 includes a valve 141‴ to aid in establishing or maintaining adequate operating vacuum pressure in the tissue resection system 100 in accordance with yet another aspect of the present disclosure. The valve 141"' is coupled between an output interface of the handpiece 142 and the fluid and debris outflow tube 158 such that the valve 141‴ is disposed outside of the surgical instrument 140 and is manually adjustable by a user. The valve 141"' is disposed within the flow path of the fluid and debris outflow tube 158 to selectively permit and inhibit flow therethrough and/or to control the flow rate therethrough. Accordingly, the valve 141"' is movable between a closed position for inhibiting flow through the fluid and debris outflow tube 158 and an open position for enabling free flow through the fluid and debris outflow tube 158. In some aspects, the valve 141‴ is a stopcock, however, other forms of valves for manually regulating fluid flow are envisioned.

In use, the valve 141‴ is positioned in the closed position while the tissue resection system 100 is not in use and during start-up to prevent the flow of ambient air through the surgical instrument 140. Once the tissue resection system 100 is primed and ready for use, the valve 141‴ is turned to the open position to enable free flow through the fluid and debris outflow tube 158. The control console 160 (FIG. 1), based on the operating status of the system 100, may provide a visual, audible, and/or other output indicating the position of the valve 141‴ and/or when the valve 141‴ should be manipulated to the other position.

As shown in FIG. 7, the handpiece 142 of the surgical instrument 140 includes a film 145 covering the interface of the handpiece 142 that connects with the end effector assembly 144 (FIG. 6). The film 145 may be applied to the handpiece 142 prior to start-up of the tissue resection system 100 (FIG. 1) by the manufacturer, after sterilization procedures, and/or in the operating room. During start-up, the handpiece 142 remains separated from the end effector assembly 144 so that the film 145 remains intact and closes off the interior of the handpiece 142 to prevent or minimize the flow of ambient air into the tissue resection system 100. It is noted that, in an initial position, the flow path through the end effector assembly 144 (FIG. 6) may also be closed, e.g., wherein the opening 155 of the cutting tool 154 is offset from the window 153 of the shaft 152 (FIG. 1), to prevent or minimize the flow of ambient air therethrough. Once the tissue resection system 100 is primed and ready for use, the film 145 is opened (e.g., pierced during attachment of the end effector assembly 144 (FIG. 6) to the handpiece 142 or removed just prior to attaching the end effector assembly 144 to the handpiece 142) to enable fluid flow through the surgical instrument 140. Accordingly, the end effector assembly 144 is attached to the handpiece 142 as one of the last steps of the start-up process.

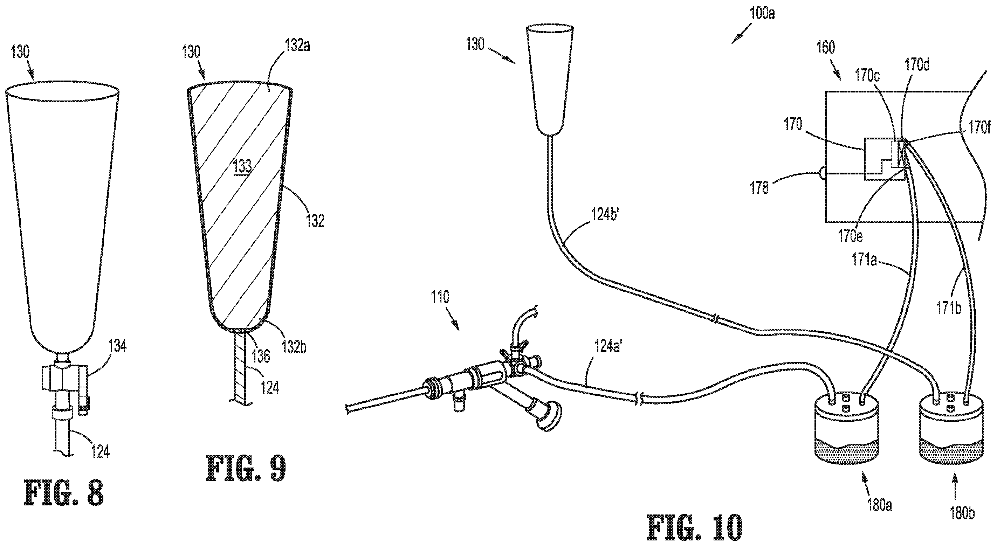

As shown in FIG. 8, the surgical drape 130 may include a valve 134 associated therewith to aid in establishing or maintaining adequate operating vacuum pressure in the tissue resection system 100 in accordance with an aspect of the present disclosure. The valve 134 is disposed between the surgical drape 130 and the fluid outflow tube 124 to control the flow of fluid and debris therethrough. The valve 134 is disposed within the flow path of the fluid outflow tube 124 to selectively permit and inhibit flow therethrough and/or to control the flow rate therethrough. Accordingly, the valve 134 is movable between a closed position for inhibiting flow through the fluid outflow tube 124 and an open position for enabling free flow through the fluid outflow tube 124.

In some aspects, the valve 134 is manually adjustable by a user. The valve 134 may be a stopcock, however, other forms of valves for manually regulating fluid flow are envisioned. In use, upon start-up of the tissue resection system 100 (FIG. 1), the valve 134 is disposed in the closed position to prevent the suctioning of ambient air through the surgical drape 130. Once the tissue resection system 100 is primed for use, the valve 134 is turned to the open position to enable flow from the surgical drape 130, through the fluid outflow tube 124, and into the collection container 180 (FIG. 1).

In other aspects, the valve 134 is controlled by the control console 160 (FIG. 2). The valve 134 may be a motorized valve that is electrically connected to the control console 160 (FIG. 2). In use, the valve 134 is closed by the control console 160 to prevent suctioning of ambient air through the surgical drape 130. Once the tissue resection system 100 (FIG. 1) is primed for use, the control console 160 sends a signal to open the valve 134 and to enable flow through the surgical drape 130 and the fluid outflow tube 124 into the collection container 180 (FIG. 1).

As shown in FIG. 9, the surgical drape 130 may include a membrane 136 extending across the cavity 133 defined in the body 132 thereof to close surgical drape 130 and prevent ambient air from being pulled therethrough. While the membrane 136 is shown as extending across the second end 132b of the body 132 of the surgical drape 130, it should be understood that the membrane 136 may extend across any portion within the cavity 133 or across the first end 132a of the body 132 so long as the membrane 136 closes the entrance of the surgical drape 130 into the fluid outflow tube 124. The membrane 136 may be formed from a dissolvable or bioerodible polymer, such as a water soluble polymer, or a biopolymer, such as sugars, starches, or salts, such that the membrane 136 dissolves, erodes, deforms, or otherwise drops off after a predetermined amount of fluid contacts the membrane 136, thereby opening the surgical drape 130 and enabling exudate to be removed therefrom, in accordance with the present claimed invention. Alternatively, the membrane 136 may be formed from any material that may pierced or removed by a user once adequate vacuum pressure is established in the tissue resection system 100.

As shown in FIG. 10, a tissue resection system 100a, which is substantially similar to the tissue resection system 100 of FIG. 1, is shown with the components necessary to illustrate the differences therebetween. The tissue resection system 100a includes first and second fluid outflow tubes 124a', 124b' for separately connecting the endoscope 110 and the surgical drape 130 to the vacuum pump assembly 170. The first fluid outflow tube 124a' couples the endoscope 110 and a first collection container 180a and the second fluid outflow tube 124b' couples the surgical drape 130 and a second collection container 180b so that vacuum may be separately drawn through the endoscope 110 and the surgical drape 130 by the vacuum pump assembly 170 of the control console 160. A first vacuum line 171a is coupled to the vacuum pump assembly 170 and the first collection container 180a for establishing negative pressure through the first collection container 180a and ultimately through the endoscope 110, and a second vacuum line 171b is coupled to the vacuum pump assembly 170 and the second collection container 180b for establishing negative pressure through the second collection container 180b and ultimately through the surgical drape 130.

The vacuum pump assembly 170 includes a vacuum line selector 170c that includes a valve 170d for controlling which of the first and second vacuum lines 171a, 171b to draw vacuum through. The vacuum line selector 170c is configured to actuate the valve 170d for drawing vacuum through only the first vacuum line 171a and thus, through the first fluid outflow tube 124a', only the second vacuum line 171b and thus, through the second fluid outflow tube 124b', both of the first and second vacuum lines 171a, 171b, or neither of the first and second vacuum lines 171a, 171b (e.g., in system configurations in which a third vacuum line is associated with a collection container coupled to a fluid and debris outflow tube of a surgical instrument).

In some aspects, the vacuum line selector 170c is coupled to an actuator 178 (e.g., a knob) disposed on the control console 160 so that a user can manually control the vacuum line selector 170c and choose which, if any, of the first and/or second vacuum lines 171a, 171b to pull vacuum through. For example, the user can choose to open the first vacuum line 171a (e.g., enable suction therethrough) and close the second vacuum line 171b (e.g., prevent suction therethrough) during start-up of the tissue section system 100a so that adequate vacuum pressure can be built up in the tissue resection system 100a for use. The user can then open both the first and second vacuum lines 171a, 171b when the tissue resection system 100a is primed for use, or leave the second vacuum line 171b closed so that exudate passed through the surgical drape 130 and the second fluid flow tube 124b' is fed into the second collection container 180b through a gravity feed.

In other aspects, the vacuum line selector 170c is motorized and programmed to automatically open and/or close the first and second vacuum lines 171a, 171b to optimize the vacuum pressure within the tissue resection system 100a. For example, the vacuum line selector 170c may be programmed to initially close the first and second vacuum lines 171a, 171b upon system start-up. As another example, the vacuum line selector 170c may be programmed to open the first vacuum line 171a when an end effector assembly 144 (FIG. 1) is attached to the handpiece 144 of the surgical instrument 140 (e.g., via RFID). In certain aspects, the vacuum pump assembly 170 may also include sensors 170e, 170f in fluid communication with each of the first and second vacuum lines 171a, 171b to monitor the flow characteristics therethrough, and the vacuum line selector 170c may be programmed to open and close the first and/or second vacuum lines 171a, 171b depending upon the flow characteristics measured by the sensors 170e, 170f (e.g., when flow characteristics are associated with air flow, the vacuum line selector 170c closes the respective first or second vacuum line 171a, 171b, and when the flow characteristics are associated with liquid flow, the vacuum line selector 170c opens the respective first or second vacuum line 171a, 171b).

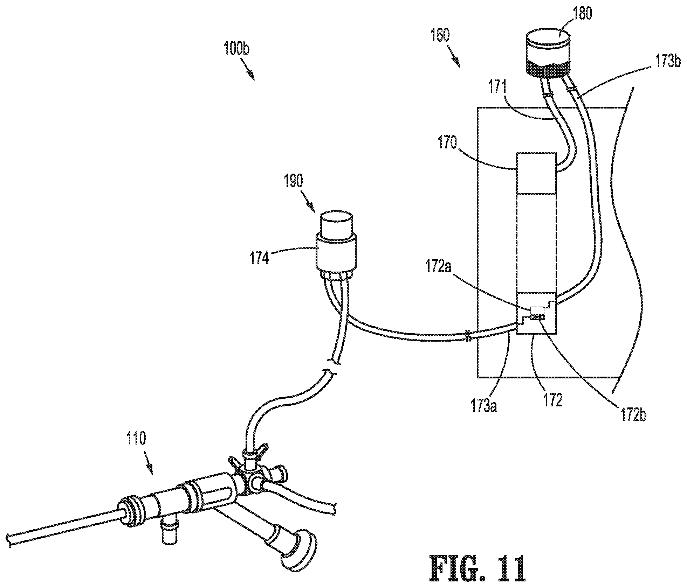

As shown in FIG. 11, a tissue resection system 100b, which is substantially similar to the tissue resection system 100 of FIG. 1 and/or the tissue resection system 100a of FIG. 10, is shown with the components necessary to illustrate the differences therebetween. The tissue resection system 100b includes first and second pressure lines 173a, 173b for separately pressurizing the fluid source 190 and the collection container 180. The first pressure line 173a couples the pressure pump assembly 172 and the fluid source 190 so that pressure may be applied to the fluid source 190 by the cuff 174 of the first pressure line 173a on the fluid source 190. The second pressure line 173b couples the pressure pump assembly 172 and the collection container 180 so that vacuum may be drawn through the collection container 180 by the pressure pump assembly 172.

The pressure pump assembly 172 includes a pressure line selector 172a that includes a valve 172b for controlling which of the first and second pressure lines 173a, 173b to utilize. The pressure line selector 172a is motorized and programmed to provide pressure through the first pressure line 173a on the fluid source 190 during use of the tissue resection system 100b. The pressure selector 172a is programmed to divert pressure from the first pressure line 173a and through the second pressure line 173b during start-up of the tissue resection system 100b to aid the vacuum pump assembly 170 in pressurizing the tissue resection system 100b. The pressure selector 172a be programmed to revert back to providing pressure only through the first pressure line 173a when the tissue resection system 100b is primed for use. In some aspects, the pressure selector 172a may be in communication with the vacuum pump assembly 170 such that if a vacuum pressure change is sensed by the vacuum pump assembly 170, the pressure selector 172a is activated to divert pressure through the second pressure line 173b to temporarily increase the vacuum capacity of the tissue resection system 100b by assisting the vacuum pump assembly 170 until adequate vacuum pressure is achieved within the tissue resection system 100b.

While aspects of the disclosure have been shown in the drawings, it is not intended that the disclosure be limited thereto, as it is intended that the disclosure be as broad in scope as the art will enable and that the specification be read likewise. It is to be understood, therefore, that the disclosure is not limited to the precise aspects described, and that various other changes and modifications may be effected by one skilled in the art without departing from the scope or spirit of the disclosure. Additionally, the elements and features shown and described in connection with certain aspects of the disclosure may be combined with the elements and features of certain other aspects without departing from the scope of the present disclosure, and that such modifications and variation are also included within the scope of the present disclosure. Therefore, the above description should not be construed as limiting, but merely as exemplifications of aspects of the disclosure. The invention is defined by the following claims.

## Claims

1. A surgical fluid management system comprising:
a surgical drape (130) including a body defining a cavity (133) therein;
a collection container (180, 180b);
a fluid outflow tube (124, 124b') coupled to the surgical drape and the collection container for withdrawal of exudate from the surgical drape into the collection container; **characterised by**
a membrane (136) extending across the cavity of the surgical drape to close a fluid flow path between the surgical drape and the fluid outflow tube wherein the membrane is formed from a dissolvable or bioerodible polymer.

2. The surgical fluid management system according to claim 1, further comprising:
a control console (160) including a vacuum pump assembly (170); and
a vacuum line (171) coupled to the vacuum pump assembly and the collection container for providing suction through the fluid outflow tube.

3. A surgical fluid management system as claimed in claim 2 comprising:
an endoscope (110);
a first collection container (180a);
a first fluid outflow tube (124a') coupled to the endoscope and the first collection container for withdrawal of fluid from the endoscope into the first collection container; and
a vacuum pump assembly (170) including a vacuum line selector (170c) for controlling suction through the outflow tubes.

4. The surgical fluid management system according to claim 3, further comprising:
a first vacuum line (171a) coupled to the vacuum pump assembly and the first collection container for providing suction through the first fluid outflow tube; and
a second vacuum line (171b) coupled to the vacuum pump assembly and the collection container (180b) for providing suction through the fluid outflow tube from the surgical drape.

## Patentansprüche

1. Chirurgisches Fluidmanagementsystem, umfassend:
ein Operationstuch (130), das einen Körper einschließt, der einen Hohlraum (133) darin definiert;
einen Sammelbehälter (180, 180b);
einen Fluidausflussschlauch (124, 124b'), der mit dem Operationstuch und dem Sammelbehälter zum Ableiten von Exsudat aus dem Operationstuch in den Sammelbehälter gekoppelt ist; **gekennzeichnet durch**
eine Membran (136), die sich über den Hohlraum des Operationstuchs erstreckt, um einen Fluidflussweg zwischen dem Operationstuch und dem Fluidausflussschlauch zu schließen, wobei die Membran aus einem löslichen oder bioerodierbaren Polymer ausgebildet ist.

2. Chirurgisches Fluidmanagementsystem nach Anspruch 1, ferner umfassend:
eine Steuerkonsole (160), die eine Vakuumpumpenanordnung (170) einschließt; und
eine Vakuumleitung (171), die mit der Vakuumpumpenanordnung und dem Sammelbehälter zum Bereitstellen einer Absaugung durch den Fluidausflussschlauch gekoppelt ist.

3. Chirurgisches Fluidmanagementsystem nach Anspruch 2, umfassend:
ein Endoskop (110);
einen ersten Sammelbehälter (180a);
einen ersten Fluidausflussschlauch (124a'), der mit dem Endoskop und dem ersten Sammelbehälter zum Ableiten von Fluid aus dem Endoskop in den ersten Sammelbehälter gekoppelt ist;
und
eine Vakuumpumpenanordnung (170), die einen Vakuumleitungswähler (170c) zum Steuern der Absaugung durch die Ausflussschläuche einschließt.

4. Chirurgisches Fluidmanagementsystem nach Anspruch 3, ferner umfassend:
eine erste Vakuumleitung (171a), die mit der Vakuumpumpenanordnung und dem ersten Sammelbehälter zum Bereitstellen der Absaugung durch den ersten Fluidausflussschlauch gekoppelt ist; und
eine zweite Vakuumleitung (171b), die mit der Vakuumpumpenanordnung und dem Sammelbehälter (180b) zum Bereitstellen der Absaugung durch den Fluidausflussschlauch von dem Operationstuch gekoppelt ist

## Revendications

1. Système de gestion de fluide chirurgical comprenant :
un champ opératoire (130) comportant un corps y définissant une cavité (133) ;
un récipient de collecte (180, 180b) ;
un tube d'écoulement de fluide (124, 124b') raccordé au champ opératoire et au récipient de collecte pour l'extraction d'exsudat du champ opératoire dans le récipient de collecte ; **caractérisé par**
une membrane (136) s'étendant sur toute la cavité du champ opératoire pour fermer une voie d'écoulement de fluide entre le champ opératoire et le tube d'écoulement de fluide, dans lequel la membrane est formée d'un polymère soluble ou bioérodable.

2. Système de gestion de fluide chirurgical selon la revendication 1, comprenant en outre :
une console de commande (160) comportant un ensemble pompe à vide (170) ; et
une conduite de vide (171) raccordée à l'ensemble pompe à vide et au récipient de collecte pour fournir une aspiration à travers le tube d'écoulement de fluide.

3. Système de gestion de fluide chirurgical selon la revendication 2, comprenant :
un endoscope (110) ;
un premier récipient de collecte (180a) ;
un premier tube d'écoulement de fluide (124a') raccordé à l'endoscope et au premier récipient de collecte pour le retrait de fluide de l'endoscope dans le premier récipient de collecte ;
et
un ensemble pompe à vide (170) comportant un sélecteur de conduite de vide (170c) permettant de réguler l'aspiration à travers les tubes d'écoulement.

4. Système de gestion de fluide chirurgical selon la revendication 3, comprenant en outre :
une première conduite de vide (171a) raccordée à l'ensemble pompe à vide et au premier récipient de collecte pour fournir une aspiration à travers le premier tube d'écoulement de fluide ; et
une seconde conduite de vide (171b) raccordée à l'ensemble pompe à vide et au récipient de collecte (180b) pour fournir une aspiration à travers le tube d'écoulement de fluide à partir du champ opératoire
